# EUROPEAN PATENT APPLICATION

(11) **EP 4 711 441 A1**
(43) Date of publication of application: **18.03.2026**
(21) Application number: 24856203.5
(22) Date of filing: 23.07.2024
(51) Int. Cl.: C12M 1/34

(54) **SENSOR ATTACHMENT MEMBER AND SENSOR UNIT PROVIDED WITH SAME**

(30) Priority: 24.08.2023 JP 2023136079
(71) Applicant: PHC HOLDINGS CORPORATION, Tokyo 100-8403 (JP)
(72) Inventor: NAKAMAE, Kenta, Toon-shi, Ehime 791-0395 (JP); TAIRA, Keisuke, Toon-shi, Ehime 791-0395 (JP); HIRATSUKA, Takashige, Toon-shi, Ehime 791-0395 (JP); FUKUMOTO, Satoshi, Toon-shi, Ehime 791-0395 (JP); WATANABE, Hidenori, Toon-shi, Ehime 791-0395 (JP)
(74) Representative: Grünecker Patent- und Rechtsanwälte PartG mbB
(86) International application number: PCT/JP2024/026269
(87) International publication number: WO 2025/041509

(57) **Abstract**

A sensor clip (10) is a member that attaches a sensor (20) used in a state of having been inserted into a flask (40) to an opening (42), and includes a mounting portion (11) and a bent portion (13). The sensor (20) has an end portion (22) including an electrode (22a), an end portion (23) including a contact (23a), a main body portion (21) that couples the end portions (22, 23), and a wiring portion (24) that is provided to the main body portion (21) and connects the electrode (22a) to the contact (23a). The main body portion (21) of the sensor (20) is mounted on the mounting portion (11). The bent portion (13) is coupled to the mounting portion (11) and is bent along the outer peripheral surface from the inner peripheral surface of the opening (42) and thereby latched to the opening (42) of the flask (40).

## Description

### TECHNICAL FIELD

The present invention relates to a sensor attachment member for attaching a sensor to a container used in a cell culture analyzer, for example, and to a sensor unit including this sensor attachment member.

### BACKGROUND ART

In recent years, cell culture analyzers have been used that measure the state of cell culture (analyze the culture state) with a measurement device placed inside an incubator (thermostat), while culturing cells with a culture module equipped with a sensor and placed in the measurement device.

With a cell culture analyzer such as this, various kinds of measurement are performed by immersing a sensor in a culture medium held in a flask of other such container.

For example, Patent Literature 1 discloses a multi-sensor component for installing sensors at individual ports of a container used for culturing biological materials.

### CITATION LIST

### PATENT LITERATURE

Patent Literature 1: JP 2021-6022 A

### SUMMARY

### (TECHNICAL PROBLEM)

However, the following problem is encountered with the conventional multi-sensor component mentioned above.

That is, the multi-sensor component disclosed in the above publication includes a housing portion that is inserted into an accommodation opening that extends through the individual ports of a container in a state in which a sensor unit is being held.

However, with this configuration, when performing a culture medium replacement operation using a pipette, for example, the housing along with the sensor unit must be removed in order to open the accommodation opening, which means that the measurement has to be interrupted, so this method is inconvenient.

It is an object of the present invention to provide a sensor attachment member with which usability can be improved by allowing tasks such as culture medium replacement to be carried out while the sensor is still attached to the opening of a container, as well as a sensor unit equipped with this sensor attachment member.

### (SOLUTION TO PROBLEM)

The sensor attachment member according to the first invention is a sensor attachment member for attaching a sensor, which is used in a state of having been inserted into a container, to an opening of the container, said sensor attachment member comprising a mounting portion and a bent portion. The sensor has a first end that includes an electrode inserted into the container, a second end that includes a contact connected to a measurement device installed outside the container, a main body portion that couples the first end and the second end, and a wiring portion that is provided to the main body portion and electrically connects the electrode and the contact. The main body portion of the sensor is mounted on the mounting portion. The bent portion is coupled to the mounting portion, is bent so as to be disposed along the outer peripheral surface from the inner peripheral surface of the opening, and is latched to the opening of the container.

Here, the sensor attachment member of the present invention is used to attach a sensor from the inside to the outside of the container through the opening in the container.

Here, the container in which the sensor is installed stores a medium for cell culture, for example, and various kinds of component are measured with this sensor. Also, the container is configured such that communication between the inside and outside is possible through the opening. The opening of the container may be configured so as to be closed off by a cap, for example.

The sensor has an electrode on the first end side that is inserted into the interior of the container, and has a contact on the second end side on the opposite side from the first end that is connected to the measurement device, and a wiring section that connects the electrode and the contact is disposed on the main body portion that couples the first end and the second end.

The mounting portion is a flat plate-shaped portion, for example, and the main body portion of the sensor is mounted thereon.

The bent portion is coupled to the mounting portion and is bent from the inner peripheral surface of the container along the outer peripheral surface, guides the main body portion of the sensor so as to be pulled out from the inside of the container to the outside, and is latched to the opening of the container.

Consequently, the sensor can be attached to the opening of the container in a state in which the opening is open, by attaching the bent portion to the opening of the container.

As a result, tasks such as replacing the culture medium can be carried out while the sensor is still attached to the opening of the container, which improves usability.

The sensor attachment member according to the second invention is the sensor attachment member according to the first invention, wherein the bent portion is latched to the opening of the container so as to sandwich the inner and outer peripheral surfaces of the opening.

Consequently, the bent portion is latched to the opening so as to sandwich the inner and outer peripheral surfaces of the container, making it possible to easily attach the sensor attachment member holding the sensor to the opening of the container.

The sensor attachment member according to the third invention is the sensor attachment member according to the first or second invention, further comprising a grip portion that is provided at the end portion of the bent portion, and is gripped by the user when attaching the sensor to the opening of the container in a state in which the sensor has been attached.

Consequently, the user can easily attach the sensor attachment member together with the sensor to the opening of the container while holding the grip portion provided at the end of the bent portion.

Also, when the sensor attachment member is attached, it is possible to prevent the user's fingers from touching the opening of the container, which effectively suppresses contamination of the inside of the container.

The sensor attachment member according to the fourth invention is the sensor attachment member according to the third invention, wherein the grip portion has a tapered surface that guides the sensor, which has been pulled out to the outside of the container through the opening, to the measurement device side in a state in which the sensor has been attached to the opening of the container.

Consequently, the sensor can be easily connected to the measurement device by guiding the main body portion of the sensor, which is pulled out from the inside to the outside of the container along the inner and outer peripheral surfaces of the opening, to the measurement device side using the tapered surface of the grip portion.

The sensor attachment member according to the fifth invention is the sensor attachment member according to the first or second invention, further comprising a holding portion that is provided along a direction intersecting the lengthwise direction of the mounting portion, and that holds the main body portion of the sensor in the lengthwise direction of the mounting portion.

The holding portion is provided so as to latch the main body portion of the sensor to the mounting portion along a direction intersecting the lengthwise direction of the mounting portion, and is one or more latching pieces, for example.

Consequently, the sensor can be attached to the opening of the container in a state in which the sensor is held with respect to the sensor attachment member.

The sensor attachment member according to the sixth invention is the sensor attachment member according to the fifth invention, wherein the holding portion includes a plurality of folded pieces that are folded back from the mounting portion in a direction intersecting the lengthwise direction of the sensor.

Consequently, the main body portion of the sensor can be held stably on the mounting portion using these folded pieces.

The sensor attachment member according to the seventh invention is the sensor attachment member according to the sixth invention, wherein the folded pieces are provided alternately on both the left and right sides in the lengthwise direction of the sensor.

Consequently, the plurality of folded pieces alternately hold the main body portion of the sensor from both the left and right sides in the lengthwise direction, which allows the main body portion of the sensor to be held more stably on the mounting portion.

The sensor attachment member according to the eighth invention is the sensor attachment member according to the first or second invention, wherein the container has a cap that closes off the opening.

Consequently, a cap that closes off the opening can be attached while the sensor is still attached to the opening with the sensor attachment member, which makes it less likely that culture medium or other such liquid inside the container will leak to the outside.

The sensor attachment member according to the ninth invention is the sensor attachment member according to the eighth invention, wherein the container has a second threaded portion formed around the outer peripheral surface of the substantially cylindrical opening, the threads of the second threaded portion meshing with a first threaded portion formed on the inner peripheral surface of the cap, and a flat portion on which these threads are not formed. The sensor is installed on the flat portion.

Consequently, the sensor can be installed on the flat portion where there is an interruption in the second threads on the outer peripheral surface of the opening, which mesh with the first threads on the inner peripheral surface of the cap, so that the cap can be put on with the sensor and sensor attachment member still attached to the opening.

The sensor attachment member according to the tenth invention is the sensor attachment member according to the first or second invention, further comprising a cutout that is formed by cutting out part of the mounting portion, and through which the first end side of the sensor placed on the mounting portion passes to the rear side of the mounting portion.

Consequently, the first end portion on the electrode side of the sensor held on the upper surface side of the mounting portion can be guided downward and disposed on the bottom side of the container.

The sensor attachment member according to the eleventh invention is the sensor attachment member according to the tenth invention, further comprising an end portion that is coupled to the mounting portion so as to sandwich the cutout, and a cushioning member that is attached so as to cover the portion of the end portion in contact with the wiring portion of the sensor.

Consequently, when the wiring portion of the sensor is passed downward through the cutout portion to attach the sensor to the sensor attachment member, the cushioning member will prevent the end portion from coming into contact with the wiring portion and damaging the sensor wiring.

The sensor unit according to the twelfth invention comprises the sensor attachment member according to the first or second invention, and the sensor that is attached to the opening of the container by the sensor attachment member.

Consequently, forming a sensor unit from a sensor attachment member and a sensor offers the same effect as above, that it is possible to carry out tasks such as replacing the culture medium while the sensor is still attached to the opening of the container, which improves usability.

The sensor unit according to the thirteenth invention is the sensor unit according to the twelfth invention, wherein the sensor has a protector that covers the wiring portion at a position running along the outer peripheral surface of the opening of the container.

Consequently, a protector that covers the wiring portion of the sensor is provided at a position running along the outer peripheral surface of the opening, which prevents the wiring portion of the sensor from being damaged by contact with the threads on the inner peripheral surface of the cap when the cap is closed.

The sensor unit according to the fourteenth invention is the sensor unit according to the thirteenth invention, wherein the protector is provided in a state of being integrated with the main body portion of the sensor so as to protrude in a direction intersecting the lengthwise direction of the main body portion, and is folded back relative to the main body portion to cover the wiring portion.

Consequently, the wiring portion can be covered by folding back the protector that is integrated with the main body portion of the sensor, so the wiring of the sensor can be protected with a simple configuration.

The sensor unit according to the fifteenth invention is the sensor unit according to the twelfth invention, wherein the sensor is used in a state in which the first end provided with the electrode has been immersed in a culture medium contained in the container, and detects components contained in the culture medium.

Consequently, the sensor can be used in a state in which the electrode provided at the first end of the sensor has been immersed in the culture medium held in a container, so the sensor can detect the various main components contained in the culture medium.

### (EFFECTS)

The sensor attachment member according to the present invention improves usability by allowing tasks such as culture medium replacement to be carried out while the sensor is still attached to the opening of the container.

### BRIEF DESCRIPTION OF THE DRAWINGS

Fig. 1 is a schematic diagram showing a state in which a sensor clip according to an embodiment of the present invention is used to place a sensor on a flask inside an incubator, and the sensor is connected to a measurement device via a contact clip;
Fig. 2A is a top view of the flask with the sensor attached via the sensor clip in Fig. 1, Fig. 2B is a side view of Fig. 2A, and Fig. 2C is a cross-sectional view along the A-A line in Fig. 2A;
Fig. 3 is a detail view showing a state in which the sensor has been attached to the opening of the flask of FIG. 2C with a sensor clip;
Fig. 4 is an oblique view of the configuration of the flask to which the sensor is attached via the sensor clip in Fig. 1;
Fig. 5A is a top view showing a state in which a cap has been attached to the opening of the flask in Fig. 4, and Fig. 5B is a cross-sectional view along the B-B line in Fig. 5A;
Fig. 6 is an oblique view of the configuration of the sensor unit including the sensor clip and sensor shown in Fig. 1, etc.;
Fig. 7 is a top view of the sensor included in the sensor unit in Fig. 6 in a state before the protector is folded back;
Fig. 8 is an oblique view showing a state in which the protector included in the sensor of Fig. 7 has been folded back; and
Fig. 9 is an oblique view of the configuration of the sensor clip for attaching the sensor shown in Fig. 8 e.t.c. to the opening of the flask.

### DESCRIPTION OF THE EMBODIMENTS

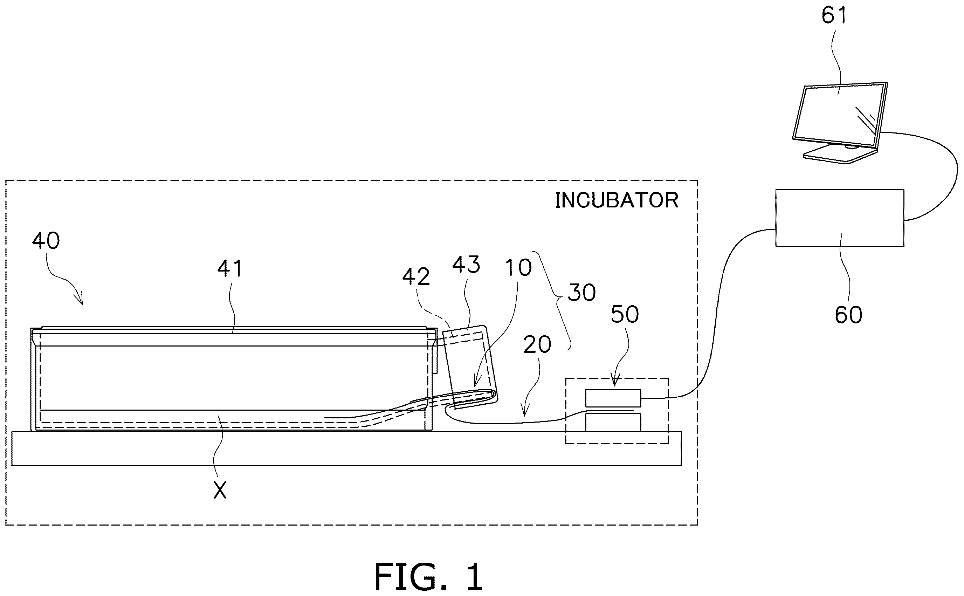

The sensor clip (sensor attachment member) 10 according to an embodiment of the present invention and a sensor unit 30 including the same will now be described with reference to Figs. 1 to 9.

In this embodiment, some unnecessarily detailed description may be omitted. For example, detailed description of already known facts or redundant description of components that are substantially the same may be omitted. This is to avoid unnecessary repetition in the following description, and facilitate an understanding on the part of a person skilled in the art.

The applicant has provided the appended drawings and the following description so that a person skilled in the art might fully understand this disclosure, but does not intend for these to limit what is discussed in the patent claims.

### (1) Configuration of Sensor Unit 30

As shown in Fig. 1, the sensor unit 30 according to this embodiment is provided with a sensor 20 that is placed in a flask (container) 40 resting on a shelf of an incubator, and detects components contained in a culture medium X while one end of the sensor is immersed in the culture medium X for cell culture, and a sensor clip 10 for attaching the sensor 20 to an opening 42 in the flask (container) 40.

As shown in Fig. 1, one end of the sensor 20 of the sensor unit 30 is immersed in the culture medium X held inside the flask 40, and the other end is connected to a measurement device 60 via a contact clip (sensor connecting member) 50 provided on the outside of the flask 40.

The measurement device 60 is a device that detects various components contained in the culture medium X using the sensor 20, for example, and is controlled by a PC (personal computer) 61.

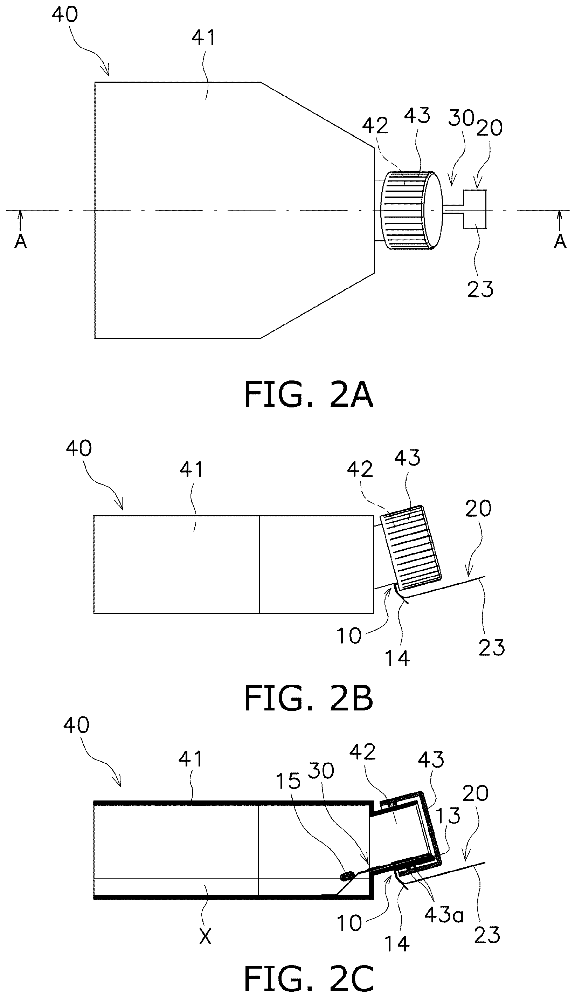

As shown in Figs. 2A, 2B, and 2C, the sensor clip 10 is used in a state of being latched to the bottom part of the opening 42 of the flask 40 in order to attach the sensor 20 to the opening 42.

The detailed configuration of the sensor clip 10 will be described later on.

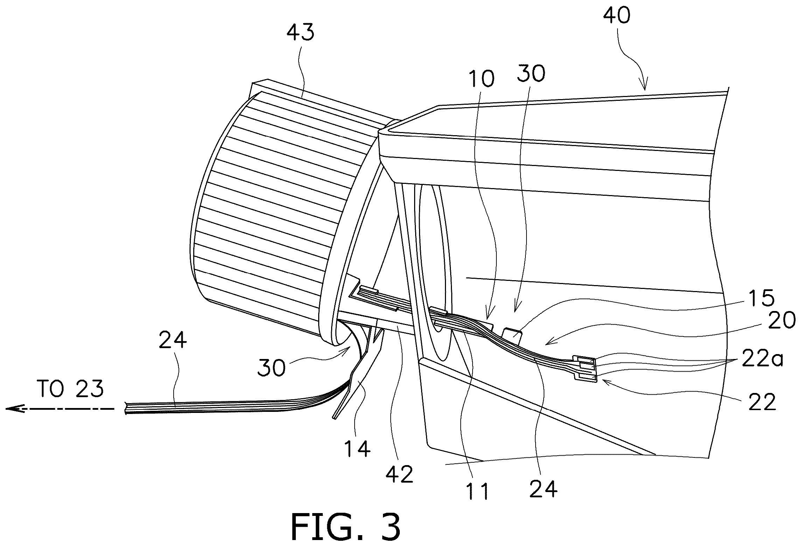

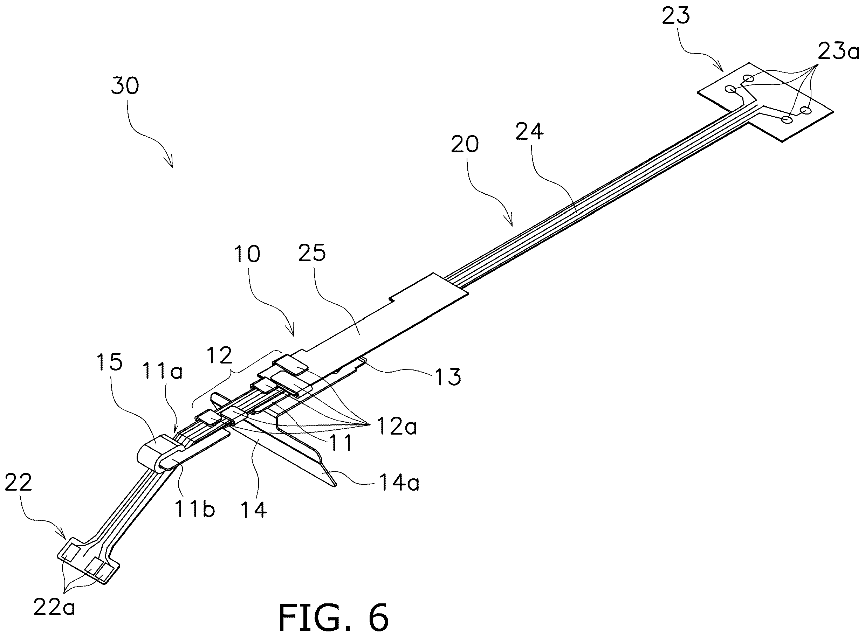

As shown in Figs. 2A, 2B, and 2C, the sensor 20 is attached to the opening 42 of the flask 40 by the sensor clip 10, and is installed so as to be pulled out from the inside of the main body portion 41 of the flask 40 to the outside through the opening 42. More precisely, as shown in Fig. 3, the sensor 20 is disposed so that the end (first end) 22 including an electrode 22a is disposed inside the flask 40, and the end (second end) 23 including a contact 23a (see Fig. 6, etc.) connected to the electrode 22a via a wiring portion 24 is pulled out to the outside of the flask 40.

Also, as shown in Figs. 2A to 3, the flask 40 is configured such that a cap 43 can be attached to the opening 42 to which the sensor 20 is attached using the sensor clip 10.

Consequently, the culture medium X, etc., can be prevented from leaking out by covering the opening 42 with the cap 43 in a state in which the sensor 20 has been attached with the sensor clip 10. Also, when replacing the culture medium X in the flask 40, for example, the cap 43 can be removed, which allows the culture medium X to be replaced using a dropper or other such tool through the opening 42 with the sensor 20 still attached.

The detailed configuration of the sensor 20 will be described below.

### (2) Configuration of Flask 40

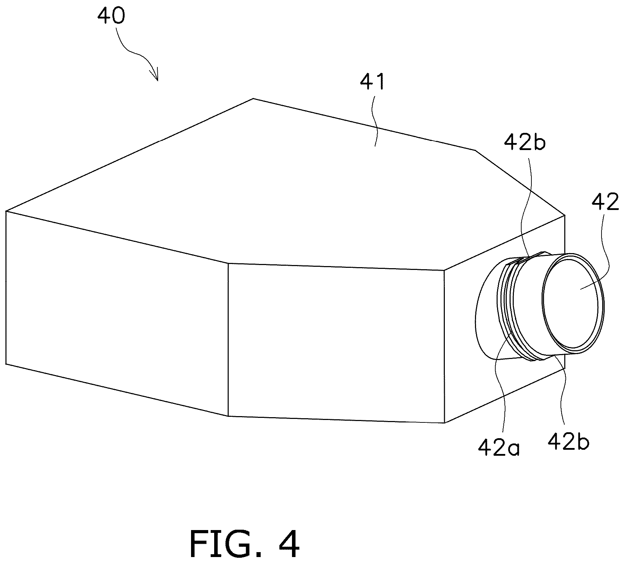

The flask (container) 40 holds the culture medium X that is used for cell culture, and as shown in Fig. 4, is a substantially rectangular container having a main body portion 41, an opening 42, and a cap 43.

As shown in Fig. 4, the main body portion 41 is a substantially rectangular housing portion in which the culture medium X is held and which is provided with the opening 42 on one of its end surfaces.

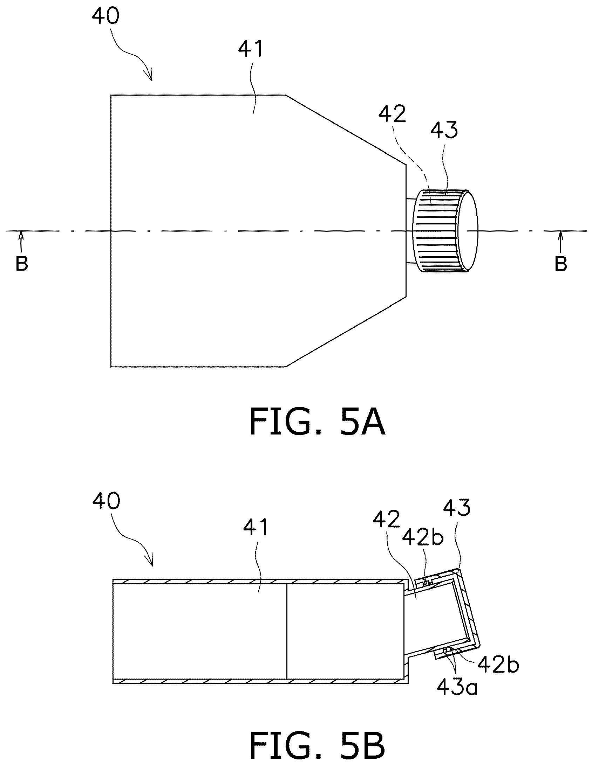

As shown in Figs. 5A and 5B, the opening 42 is a substantially cylindrical portion that is provided to the end surface of the substantially rectangular main body portion 41, allowing the inside and outside of the main body portion 41 to communicate, and is provided facing obliquely upward from the inside to the outside of the main body portion 41. As shown in Fig. 4, the opening 42 has threads (second threaded portion) 42a and flat portions 42b on its outer peripheral surface.

As shown in Fig. 4, the threads (second threaded portion) 42a are provided on the outer peripheral surface of the substantially cylindrical opening 42, and mesh with threads (first threaded portion) 43a on the cap 43 side.

As shown in Fig. 4, the flat portions 42b are provided on the upper and lower parts of the outer peripheral surface of the substantially cylindrical opening 42, and are flat surface portions where the threads 42a are partially interrupted. When the sensor 20 is attached to the opening 42 of the flask 40 by the sensor clip 10, the portion of the sensor 20 covered by the protector 25 is disposed on the flat portions 42b (see Fig. 2C).

Consequently, even when cap 43 is placed over the opening 42, the sensor 20 is disposed together with sensor clip 10 in the gap formed between the inner peripheral surface of the cap 43 and the outer peripheral surface of the opening 42. Therefore, even in a state in which sensor 20 has been attached to the opening 42 using the sensor clip 10, the cap 43 that closes off the opening 42 can be put in place.

The cap 43 is a cylindrical lid member with a bottom that closes off the opening 42, and as shown in Fig. 5B, has threads (first threaded portion) 43a on its inner peripheral surface.

As shown in Fig. 5B, the threads (first threaded portion) 43a are provided on the inner surface side of the cylindrical cap 43, and mesh with the threads 42a (see Fig. 4) on the opening 42 side, thereby closing off the opening 42 with the cap 43.

### (3) Configuration of Sensor 20

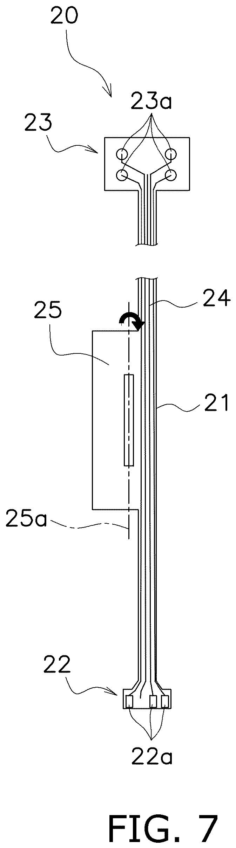

As shown in Fig. 6, the sensor 20 is included in the sensor unit 30, and is attached to the opening 42 of the flask 40 in a state of being held by the sensor clip 10. As shown in Fig. 7, the sensor 20 has an end portion (first end portion) 22, an end portion (second end portion) 23, a main body portion 21 that couples the end portions 22 and 23, a wiring portion 24, and a protector 25.

The main body portion 21 is a thin plate-like member molded from polyethylene terephthalate (PET) resin, for example, and the end portion 22 including an electrode 22a is provided at one end in the lengthwise direction, and the end portion 23 including a contact 23a is provided at the other end, as shown in Fig. 7. Also, the protector 25 is provided so as to protrude in a direction intersecting the lengthwise direction of the main body portion 21.

As shown in Fig. 7, the electrode 22a, which is inserted into the flask 40, is provided at the end portion (first end portion) 22. The end portion 22 is disposed so that the electrode 22a is immersed in the culture medium X held in the flask 40.

As shown in Fig. 7, the end portion (second end portion) 23 is provided with the contact 23a that is connected to a measurement device 60 installed on the outside of the flask 40. The contact 23a of the end portion 23 is connected to the measurement device 60 installed outside the incubator, via a contact clip 50 (see Fig. 1).

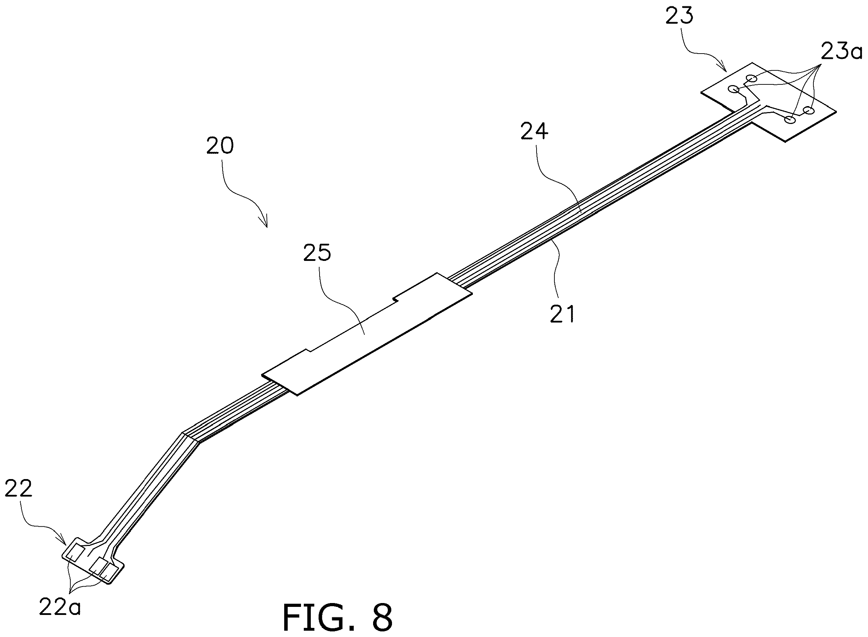

As shown in Figs. 7 and 8, the wiring portion 24 is provided along the upper surface of the main body portion 21, from the end portion 22 to the end portion 23, and electrically connects the electrode 22a and the contact 23a.

The protector 25 is a thin plate-like member molded from PET resin, as is the main body portion 21, and is coupled to the main body portion 21 in a direction intersecting the lengthwise direction. The protector 25 is folded back from the main body portion 21 in the direction of the arrow shown in Fig. 7 at a folding line 25a provided at the portion coupled with the main body portion 21, thereby covering a part of the wiring portion 24 provided on the upper surface of the main body portion 21 as shown in Fig. 8.

Here, the portion of the wiring portion 24 covered by the protector 25 shown in Fig. 8 is disposed on the outer peripheral surface of the opening 42 in a state in which the sensor 20 has been attached to the opening 42 of the flask 40 shown in Fig. 3, etc.

Consequently, when the sensor 20 is attached to the opening 42 of the flask 40 with the sensor clip 10, the protector 25 prevents the wiring portion 24 of the sensor 20 from being damaged by the threads 43a provided on the inner surface of the cap 43, for example.

### (4) Configuration of Sensor Clip 10

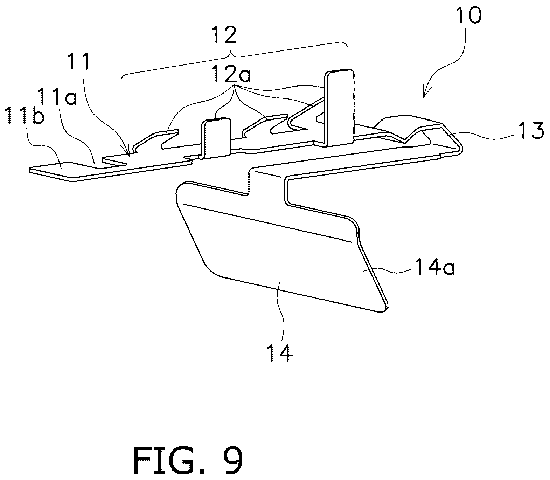

The sensor clip (sensor attachment member) 10 in this embodiment is formed by sheet metal working of a stainless steel sheet, for example, and as shown in Fig. 3, etc., the sensor 20 is attached to the opening 42 of the flask 40 in a state of being held by the sensor clip 10. As shown in Fig. 9, the sensor clip 10 includes a mounting portion 11, a holding portion 12, a bent portion 13, a grip portion 14, and a silicone tube (cushioning member) 15.

The mounting portion 11 is a long, thin plate-like portion on the upper surface of which the sensor 20 is mounted. As shown in Fig. 9, an end portion 11b is at one end of the mounting portion 11, and the bent portion 13 and the grip portion 14 are coupled to the other end. Also, as shown in Fig. 9, a cutout portion 11a formed by cutting out part of the mounting portion 11 is provided between the mounting portion 11 and the end portion 11b.

As shown in Fig. 9, the cutout portion 11a is provided by cutting out part of the mounting portion 11, and allows the end portion 22 side including the electrode 22a of the sensor 20 mounted on the upper surface of the mounting portion 11 to be passed through to the back side of the mounting portion 11. Consequently, the end portion 22 including the electrode 22a of the sensor 20 is guided from the upper surface side to the back side of the mounting portion 11 and is immersed in the culture medium X held in the flask 40.

As shown in Fig. 9, the end portion 11b is coupled to the mounting portion 11 so as to sandwich the cutout portion 11a in the same plane as the mounting portion 11. More precisely, the end portion 11b is coupled to the mounting portion 11 by a narrow portion excluding the part where the cutout portion 11a is provided.

The holding portion 12 is provided in a direction intersecting the lengthwise direction of the mounting portion 11, and holds the main body portion 21 of the sensor 20 along the lengthwise direction on the upper surface of the mounting portion 11 (see Fig. 6 ). As shown in Fig. 9, the holding portion 12 has a plurality of folded pieces 12a that are folded back from the mounting portion 11 in a direction intersecting the lengthwise direction of the sensor 20.

As shown in Fig. 9, the folded pieces 12a are provided alternately on both the left and right sides in the lengthwise direction of the sensor 20. Consequently, the sensor 20 can be securely held from both sides on the upper surface of the mounting portion 11 using the folded pieces 12a, as shown in Fig. 6, etc.

As shown in Fig. 9, the bent portion 13 is coupled to the mounting portion 11, is bent so as to be disposed from the inner peripheral surface to the outer peripheral surface of the opening 42 of the flask 40, and is latched to the opening 42 of the flask 40. The bent portion 13 is attached to the opening 42 so as to sandwich the inner and outer peripheral surfaces of the opening 42 of the flask 40 (see Fig. 2C).

Consequently, the sensor clip 10 can be easily attached to the opening 42 of the flask 40 while holding the sensor 20.

As shown in Fig. 9, the grip portion 14 is provided at the end of the bent portion 13 on the opposite side from the end coupled to the mounting portion 11. The grip portion 14 is gripped by the user's fingers when attaching the sensor 20 to the opening 42 of the flask 40.

Consequently, in attaching the sensor clip 10 to the opening 42 of the flask 40 while the sensor 20 is being held, this is performed in a state of gripping the grip portion 14, which is provided as far away as possible from the bent portion 13 latched to the opening 42. This prevents the user's fingers from accidentally touching the area around the opening 42, and makes contamination inside the flask 40 through the opening 42 less likely to occur.

Also, as shown in Fig. 9, the grip portion 14 has a tapered surface 14a that is bent at a specific angle with respect to the upper surface of the grip portion 14 that is coupled to the bent portion 13.

In a state in which the sensor 20 has been attached to the opening 42 of the flask 40, the tapered surface 14a guides the end portion 23 including the contact 23a so that the sensor 20, which has been pulled out of the flask 40 through the opening 42, is folded back toward the measurement device 60 from a state of having been bent along the bent portion 13.

The silicone tube (cushioning member) 15 is a cushioning member made of a silicone resin, for example, and is attached so as to cover the contact portion with the wiring portion 24 of the sensor 20 at the end portion 11b, as shown in Fig. 6.

This prevents the wiring portion 24 of the sensor 20, which passes through the cutout portion 11a and is guided from the upper surface of the mounting portion 11 to the rear surface side, from touching the end portion 11b and thereby being damaged.

### Main Features

As shown in Fig. 3, etc., the sensor clip 10 of this embodiment is a member used for attaching the sensor 20, which is used in a state of having been inserted into the flask 40, to the opening 42, and is provided with the mounting portion 11 and the bent portion 13. The sensor 20 has the end portion 22 including the electrode 22a to be inserted inside the flask 40, the end portion 23 including the contact 23a to be coupled to a measurement device 60 installed outside the flask 40, the main body portion 21 coupling the end portions 22 and 23, and the wiring portion 24 that is provided to the main body portion 21 and electrically connects the electrode 22a and the contact 23a. The main body portion 21 of the sensor 20 is mounted on the mounting portion 11. The bent portion 13 is coupled to the mounting portion 11 and is bent so as to be disposed along the outer peripheral surface from the inner peripheral surface of the opening 42, and is latched to the opening 42 of the flask 40.

Consequently, the sensor 20 can be attached to the opening 42 of the flask 40 while keeping the opening 42 open by attaching the bent portion to the opening 42 of the flask 40.

As a result, replacement of the culture medium X, etc., can be carried out while the sensor 20 is still attached to the opening 42 of the flask 40, which improves usability.

### Other Embodiments

An embodiment of the present invention was given above, but the present invention is not limited to or by the above embodiment, and various modifications are possible without departing from the gist of the invention.
(A)

In the above embodiment, an example was given in which the sensor clip 10 was used to attach the sensor 20, which was used in a state in which the end portion 22 including the electrode 22a had been immersed in the culture medium X used for cell culture, to a container such as the flask 40. However, the present invention is not limited to this.

For example, the sensor attachment member (sensor clip) is not limited to sensors used in cell culture, and may be used to attach various kinds of sensor to a container.
(B)

In the above embodiment, an example was given in which the protector 25 that covered and protected the wiring portion 24 of the sensor 20 was used by folding back a part of the main body portion 21. However, the present invention is not limited to this.

For example, the configuration may instead be such that a protector that covers the wiring portion of a sensor is provided as a separate member from the main body portion of the sensor.
(C)

In the above embodiment, an example was given in which the sensor clip 10 was configured so that the grip portion 14, which was gripped by the user's fingers, was provided facing downward at the end of the bent portion 13. However, the present invention is not limited to this.

For example, the grip portion for gripping the sensor attachment member may be configured to protrude laterally from the end of the bent portion, or may be provided at some other position.
(D)

In the above embodiment, an example was given in which a plurality of the folded pieces 12a were used as the holding portion 12 that held the main body portion 21 of the sensor 20 in the mounting portion 11 of the sensor clip 10. However, the present invention is not limited to this.

For example, the configuration may be such that the main body portion of the sensor is held on the mounting portion of the sensor attachment member by a single folded piece.

Also, the holding portion may be configured to be able to fix the main body portion of the sensor to the mounting portion.

For example, the back surface of the sensor and the mounting portion may be affixed and held together using double-sided adhesive tape. Alternatively, the sensor and mounting portion may be put together with tape, and tape then wrapped around the outer periphery to hold them together. Also, the sensor and mounting portion may be sandwiched and held together by some means such as an ordinary clip. Also, the sensor and mounting portion may be put together and then sandwiched between two plate-like members, and the two plate-like members may be fixed with fastening members such as screws to hold them together.
(E)

In the above embodiment, an example was given in which a plurality of folded pieces 12a were provided alternately on the left and right sides in the lengthwise direction of the sensor 20, as the holding portion 12 that holds the main body portion 21 of the sensor 20 in the mounting portion 11 of the sensor clip 10. However, the present invention is not limited to this.

For example, the configuration may be such that the folded pieces are provided on the same side in the lengthwise direction of the sensor, or are provided randomly rather than alternately.
(F)

In the above embodiment, an example was given in which the opening 42 of the flask 40 to which the sensor 20 was attached using the sensor clip 10 was closed off with the cap 43. However, the present invention is not limited to this.

For example, the configuration may be such that the sensor is attached via a sensor attachment member to the opening of a container that has no cap.
(G)

In the above embodiment, an example was given in which the flat portion 42b having no threads was provided to a part of the outer peripheral surface of the opening 42 of the flask 40 to which the sensor 20 was attached using the sensor clip 10. However, the present invention is not limited to this.

For example, the configuration may be such that the sensor is attached via a sensor attachment member to a threaded opening formed all the way around the outer peripheral surface in the peripheral direction.

With this configuration, if a cap is to be placed over the opening, a cap with a slightly larger inner diameter than usual should be used.

### INDUSTRIAL APPLICABILITY

The sensor attachment member of the present invention exhibits the effect that usability can be improved by carrying out tasks such as culture medium replacement while the sensor is still attached to the opening of the container, and therefore can be broadly applied to analysis devices used, for example, with cell culture devices that perform various kinds of measurement.

### REFERENCE SIGNS LIST

10 sensor clip (sensor attachment member)
11 mounting portion
11a cutout portion
11b end portion
12 holding portion
12a folded piece
13 bent portion
14 grip portion
14a tapered surface
15 silicone tube (cushioning material)
20 sensor
21 main body portion
22 end portion (first end portion)
22a electrode
23 end portion (second end portion)
23a contact
24 wiring portion
25 protector
25a folding line
30 sensor unit
40 flask (container)
41 main body portion
42 opening
42a thread (second threaded portion)
42b flat portion
43 cap
43a thread (first threaded portion)
50 contact clip (sensor connecting member)
60 measurement device
61 PC
X culture medium

## Claims

1. A sensor attachment member for attaching a sensor, which is used in a state of having been inserted into a container, to an opening of the container,:
the sensor has a first end that includes an electrode inserted into the container, a second end that includes a contact connected to a measurement device installed outside the container, a main body portion that couples the first end and the second end, and a wiring portion that is provided to the main body portion and electrically connects the electrode and the contact, and
the sensor attachment member comprising;
a mounting portion on which the main body portion of the sensor is mounted; and
a bent portion that is coupled to the mounting portion, is bent so as to be disposed along an outer peripheral surface from an inner peripheral surface of the opening, and is latched to the opening of the container.

2. The sensor attachment member according to claim 1,
wherein the bent portion is latched to the opening of the container so as to sandwich the inner peripheral surface and outer peripheral surface of the opening.

3. The sensor attachment member according to claim 1 or 2,
further comprising a grip portion that is provided at an end portion of the bent portion, and is gripped by a user when attaching the sensor to the opening of the container in a state in which the sensor has been attached.

4. The sensor attachment member according to claim 3,
wherein the grip portion has a tapered surface that guides the sensor, which has been pulled out to an outside of the container through the opening, to a side of the measurement device in a state in which the sensor has been attached to the opening of the container.

5. The sensor attachment member according to claim 1 or 2,
further comprising a holding portion that is provided along a direction intersecting a lengthwise direction of the mounting portion, and that holds the main body portion of the sensor in the lengthwise direction of the mounting portion.

6. The sensor attachment member according to claim 5,
wherein the holding portion includes a plurality of folded pieces that are folded back from the mounting portion in a direction intersecting the lengthwise direction of the sensor.

7. The sensor attachment member according to claim 6,
wherein the folded pieces are provided alternately on both left and right sides in the lengthwise direction of the sensor.

8. The sensor attachment member according to claim 1 or 2,
wherein the container has a cap that closes off the opening.

9. The sensor attachment member according to claim 8,
wherein the container has a second threaded portion formed around the outer peripheral surface of the substantially cylindrical opening, the threads of the second threaded portion meshing with a first threaded portion formed on the inner peripheral surface of the cap, and a flat portion on which these threads are not formed, and
the sensor is installed on the flat portion.

10. The sensor attachment member according to claim 1 or 2,
further comprising a cutout that is formed by cutting out part of the mounting portion, and through which a side of the first end of the sensor placed on the mounting portion passes to a rear side of the mounting portion.

11. The sensor attachment member according to claim 10, further comprising:
an end portion that is coupled to the mounting portion so as to sandwich the cutout; and
a cushioning member that is attached so as to cover the portion of the end portion in contact with the wiring portion of the sensor.

12. A sensor unit, comprising:
the sensor attachment member according to claim 1 or 2; and
the sensor that is attached to the opening of the container by the sensor attachment member.

13. The sensor unit according to claim 12,
wherein the sensor has a protector that covers the wiring portion at a position running along the outer peripheral surface of the opening of the container.

14. The sensor unit according to claim 13,
wherein the protector is provided in a state of being integrated with the main body portion of the sensor so as to protrude in a direction intersecting a lengthwise direction of the main body portion, and is folded back relative to the main body portion to cover the wiring portion.

15. The sensor unit according to claim 12,
wherein the sensor is used in a state in which the first end provided with the electrode has been immersed in a culture medium contained in the container, and detects components contained in the culture medium.
